# EUROPÄISCHE PATENTSCHRIFT

(11) **EP 2 384 700 B1**
(45) Veröffentlichungstag und Bekanntmachung des Hinweises auf die Patenterteilung: **01.04.2015**
(21) Anmeldenummer: 11164484.5
(22) Anmeldetag: 02.05.2011
(51) Int. Cl.: A61B 6/00, A61B 6/03, A61B 6/10, H05G 1/02

(54) **Tragbare Computertomographievorrichtung**
Portable computer tomography device
Dispositif de tomographie assistée par ordinateur portative

(30) Priorität: 03.05.2010 DE 102010028511
(43) Veröffentlichungstag der Anmeldung: 09.11.2011
(73) Patentinhaber: Fraunhofer-Gesellschaft zur Förderung der angewandten Forschung e.V., 80686 München (DE); Friedrich-Alexander-Universität Erlangen-Nürnberg, 91054 Erlangen (DE)
(72) Erfinder: Hebele, Stefan, 91785, Mischelbach (DE); Nachtrab, Frank, 91058, Erlangen (DE); Uhlmann, Norman, 91056, Erlangen (DE); Hanke, Randolf, 90617, Puschendorf (DE); Burtzlaff, Susanne, 90762 Fürth (DE); Sukowski, Frank, 92353 Postbauer-Heng (DE); Salamon, Michael, 90763, Fürth (DE)
(74) Vertreter: Stöckeler, Ferdinand

(56) Entgegenhaltungen:
- WO-A2-2007/106419
- US-A- 5 610 968
- US-A1- 2004 218 716
- US-A1- 2006 140 345
- US-A1- 2008 078 940
- US-B2- 7 684 544

## Beschreibung

Die vorliegende Erfindung bezieht sich auf eine Computertomographievorrichtung und insbesondere eine tragbare Computertomographievorrichtung.

Computertomographievorrichtungen ermöglichen eine Aufnahme vieler Röntgenbilder eines Objekts aus verschiedenen Richtungen, um basierend auf diesen Röntgenbildern eine dreidimensionale Darstellung des Objekts zu erzeugen. Bekannte Computertomographieanlagen sind zu groß, schwer und unhandlich, um von einer Person getragen werden zu können bzw. dürfen. Diesbezüglich begrenzt der Arbeitsschutz in Deutschland das maximale Gewicht tragbarer Gegenstände auf 25 kg.

Es sind transportierbare Computertomographieanlagen bekannt, bei denen eine Transportmöglichkeit über Gestelle mit Rädern realisiert wird. Gewichte dieser Anlagen reichen von 80 kg bis 32 kg. Um die nötige Steifigkeit zu erhalten, wird Stahl als Grundelement verwendet. Bei solchen bekannten transportablen Systemen ist das gesamte Gehäuse von einer Abschirmung umgeben. Derartige transportable Systeme sind jedoch aufgrund ihres Gewichts und ihrer äußeren Abmaße nicht als tragbare Systeme geeignet.

Aus der US 2004/0218716 A1 ist ein als tragbar bezeichnetes Bilderzeugungssystem bekannt, bei dem eine Röntgenquelle und ein Röntgendetektor in einem Gehäuse relativ zu einem Objekt verfahrbar sind. In dem Bilderzeugungsystem sind nur einige Komponenten abgeschirmt.

Aus der US 7684544 B2 ist ein Handröntgengerät bekannt, das einen Röntgenstrahlgenerator, Touchscreen-Schnittstellen, eine Synchronisationsschaltung und ein Computersystem, die Teile einer einzelnen Einheit sind, aufweist.

Die WO 2007/106419 A2 offenbart eine Tomographievorrichtung, die ein Trägergestell, eine Positionierungseinrichtung für das Trägergestell, eine Röntgenstrahlquelle, die an einem Trägerarm angebracht ist, der relative zu dem Trägergestell beweglich ist, und einen Röntgenstrahldetektor aufweist.

Die Aufgabe der vorliegenden Erfindung besteht darin, eine tragbare Computertomographievorrichtung zu schaffen.

Diese Aufgabe wird durch eine Computertomographievorrichtung nach Anspruch 1 gelöst.

Die vorliegende Erfindung basiert auf der Erkenntnis, dass es nicht notwendig ist, bei einer Computertomographievorrichtung sämtliche Komponenten innerhalb einer Abschirmung anzuordnen, sondern dass es ausreicht, die Komponenten, die der Röntgenstrahlung ausgesetzt sind, in dem abgeschirmten Raum anzuordnen. Durch eine solche Reduzierung des abgeschirmten Raumes kann das Abschirmmaterial reduziert werden, und folglich das Gewicht der Computertomographievorrichtung.

Bei Ausführungsbeispielen der Erfindung werden nur eine Röntgenstrahlungsquelle, ein Objektraum und der Teil eines Röntgenstrahlungsdetektors, der Röntgenstrahlung ausgesetzt ist, mit einer Abschirmung versehen, während andere Komponenten, wie z. B. ein Hochspannungsgenerator oder Steuereinrichtungen für eine oder mehrere Positionierungseinrichtungen nicht mit einer Abschirmung versehen sind.

Ausführungsbeispiele der Erfindung weisen eine Einrichtung zum Kühlen der Röntgenstrahlungsquelle auf, die teilweise oder ganz in dem abgeschirmten Raum angeordnet sein kann. Durch das Vorsehen einer Kühleinrichtung ist es möglich, die Abmessungen der Computertomographievorrichtung zu reduzieren, so dass diese in einem tragbaren Gehäuse implementiert werden kann.

Bei Ausführungsbeispielen der Erfindung weist die Einrichtung zum Kühlen Wärnekoppelelemente, die an der Röntgenstrahlungsquelle angebracht sind, Wärmeleitungen zum Ableiten von Wärme von den Wärmekoppelelementen zu Kühllamellen und zumindest einen Lüfter, um Wärme von den Kühllamellen nach außen zu transportieren, auf. Eine solche Kühleinrichtung ermöglicht einen kompakten Aufbau der Computertomographievorrichtung.

Ausführungsbeispiele der Erfindung umfassen ferner eine Positionierungseinrichtung zum Positionieren eines Objekts in dem Objektraum, wobei eine Rotationseinrichtung vorgesehen sein kann, um ein Objekt relativ zu der durch die Röntgenstrahlungsquelle erzeugten Strahlung zu drehen. Alternativ oder zusätzlich kann eine Verfahreinrichtung vorgesehen sein, die ausgelegt ist, um das Objekt relativ zu der Röntgenstrahlung zu verfahren, um verschiedene Abschnitte des Objekts der Röntgenstrahlung auszusetzen. Somit ermöglichen Ausführungsbeispiele der vorliegenden Erfindung die Erzeugung einer Schrauben-Computertomographie (Helix-Computertomographie).

Bei Ausführungsbeispielen der Erfindung sind elektronische Bauteile der Computertomographievorrichtung außerhalb des abgeschirmten Raums angeordnet. Dadurch können die elektronischen Bauteile durch die Abschirmung vor der Röntgenstrahlung geschützt werden. Beispiele solcher elektronischen Bauteile sind ein oder mehrere Steuerungen für die Positionierungseinrichtung.

Bei Ausführungsbeispielen der vorliegenden Erfindung weisen eine Röntgenstrahlungsquelle und ein Hochspannungsgenerator eine Längsachse auf, wobei die Röntgenstrahlungsquelle und der Hochspannungsgenerator derart angeordnet sind, dass die Längsachsen derselben senkrecht zueinander sind. Somit ist es möglich, diese Komponenten auf Platz sparende Weise anzuordnen. Ferner kann durch eine solche Vorgehensweise der Schwerpunkt der Computertomographievorrichtung mehr zur Mitte hin verschoben werden, was positiv hinsichtlich der Tragbarkeit der Computertomographievorrichtung ist.

Bei Ausführungsbeispielen der vorliegenden Erfindung können Wände des tragbaren Gehäuses in Sandwich-Bauweise ausgeführt sein. Eine solche Bauweise eignet sich insbesondere für eine Bodenplatte des tragbaren Gehäuses, da dadurch eine hohe Festigkeit bei geringem Gewicht erreicht werden kann.

Ausführungsbeispiele der vorliegenden Erfindung weisen ein Schienensystem auf, an dem Röntgenstrahlungsquelle, Positionierungseinrichtung und Röntgenstrahlendetektor befestigt sind. Dadurch kann eine hochgenaue Ausrichtung der genannten Komponenten zueinander erreicht werden, so dass die zur Erzeugung einer Computertomographie erforderlichen Signale auf vorteilhafte Weise erhalten werden können. Bei Ausführungsbeispielen der Erfindung umfasst die Computertomographievorrichtung einen USB-Anschluss, so dass ein kompliziertes Anschließen der Anlage an einen Computer entfallen kann. Über den USB-Anschluss können die erzeugten Signale an den Computer ausgegeben werden, der dann die zur Erzeugung der Computertomographie-Darstellung erforderlichen Berechnungen durchführen kann. Bei alternativen Ausführungsbeispielen kann die Computertomographievorrichtung auch Rechenmittel aufweisen, um die erforderlichen Berechnungen teilweise oder ganz durchzuführen. Bei wiederum alternativen Ausführungsbeispielen kann die Computertomographievorrichtung auch einen Bildschirm aufweisen, der eine Anzeige der aufgenommenen Bilder ermöglicht.

Unter einem "tragbaren" Gehäuse wird hierin ein solches verstanden, das Abmessungen aufweist, die es einer Person ermöglichen, die Computertomographievorrichtung zu tragen. Unter einer "tragbaren" Computertomographievorrichtung wird dabei bei Ausführungsbeispielen der Erfindung eine Computertomographievorrichtung verstanden, deren Gesamtgewicht maximal 25 kg beträgt, d. h. das Gewicht, auf das das maximal tragbare Gewicht durch den Arbeitsschutz begrenzt ist. Alternative Ausführungsbeispiele der Erfindung können ein Gewicht von weniger als 20 kg aufweisen. Ausführungsbeispiele erfindungsgemäßer Computertomographievorrichtungen können eine Länge von weniger als 500 mm, eine Breite von weniger als 500 mm und eine Höhe von weniger als 300 mm aufweisen. Ein von den Erfindern hergestellter Prototyp einer Computertomographievorrichtung wies ein Gewicht von 19 kg, eine Länge von 350 mm, eine Breite von 300 mm und eine Höhe von 230 mm auf.

Ausführungsbeispiele der Erfindung schaffen somit einen tragbaren Computertomographen, der von einer Person tragbar ist, indem er kompakte Abmessungen aufweist, eine Helix-Computertomographie ermöglicht, eine innovative Kühlung für eine Röntgenstrahlungsquelle aufweist, eine gewichtsreduzierte Abschirmung besitzt und in Leichtbaukonstruktion ausgeführt ist.

Ausführungsbeispiele der vorliegenden Erfindung werden nachfolgend anhand der beigefügen Zeichnungen näher erläutert. Es zeigen:
- Fig.1a,1b: zwei gegeneinander um 180° gedrehte perspektivische Ansichten von Gehäuseteilen und einer Abschirmungseinrichtung eines Ausführungsbeispiels einer erfindungsgemäßen Computertomographievorrichtung;
- Fig. 2: schematisch eine Draufsicht auf Merkmale eines Ausführungsbeispiels einer erfindungsgemäßen Computertomographievorrichtung;
- Fig. 3: schematisch eine Bodenplatte mit Schienensystem eines Ausführungsbeispiels einer erfindungsgemäßen Computertomographievorrichtung;
- Fig. 4: schematisch eine Röntgenstrahlungsquelle mit einer Kühlungseinrichtung; und
- Fig. 5: schematisch eine Seitenansicht der Röntgenstrahlungsquelle mit einer Kühlungseinrichtung.

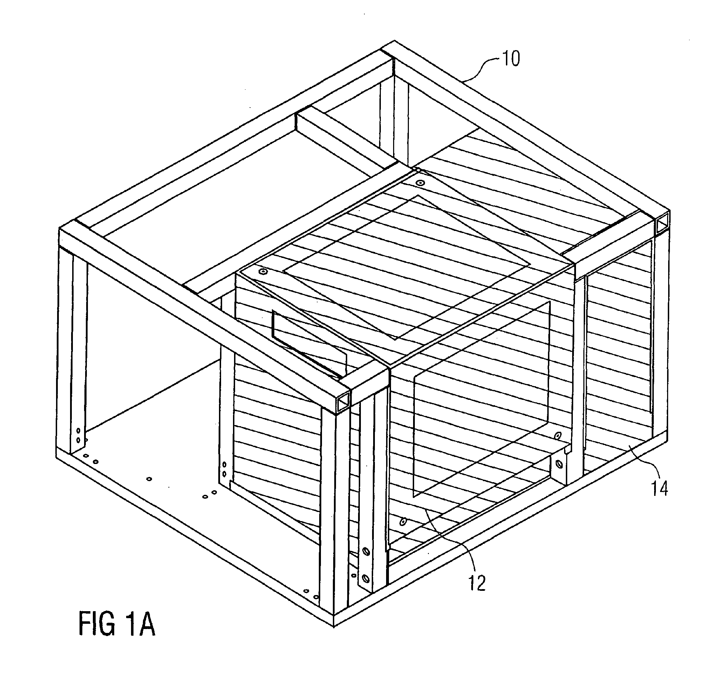

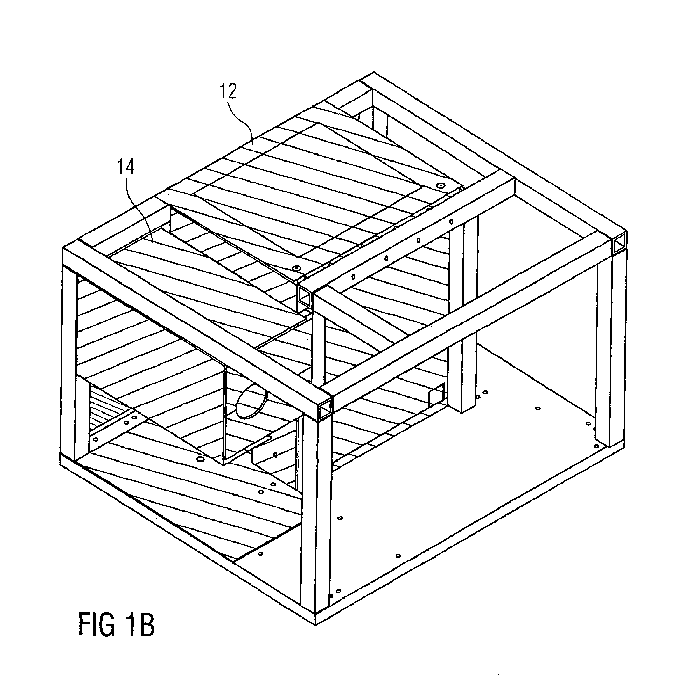

Figuren 1a und 1b zeigen schematisch eine Rahmenstruktur 10 eines tragbaren Gehäuses eines Ausführungsbeispiels einer erfindungsgemäßen Computertomographievorrichtung. Wie dargestellt ist, kann die Rahmenstruktur 10 beispielsweise aus hohlen quadratischen Leisten hergestellt sein, die beispielsweise aus Aluminium bestehen können. Die Rahmenstruktur 10 kann mit äußeren Verkleidungen, die jedoch nicht zum Zwecke der Abschirmung einer Röntgenstrahlung dienen, versehen sein. Beispielsweise können die äußeren Abdeckungen aus Kunststoff bestehen. Die äußeren Abdeckungen können teilweise entfernbar, wie z. B. aufklappbar sein, um bei Bedarf einen Zugriff auf innere Komponenten der Computertomographievorrichtung zu ermöglichen. Die äußeren Abdeckungen sind in den Figuren 1 a und 1b nicht gezeigt.

An der Rahmenstruktur 10 sind Abschirmungen 12 und 14 auf geeignete Weise befestigt, wie in den Figuren 1a und 1b gezeigt ist. Die Abschirmungen 12 und 14 sind vorgesehen, um die Bereiche der Computertomographievorrichtung, in denen Röntgenstrahlung auftritt, gegen die Umgebung abzuschirmen. Wie die nachfolgenden Erörterungen zeigen werden, ist die Abschirmung 12 vorgesehen, um einen Objektraum, der auch als Probenraum bezeichnet werden kann, und zumindest einen Teil eines Röntgenstrahlendetektors abzuschirmen, während die Abschirmung 14 vorgesehen ist, um eine Röntgenstrahlungsquelle abzuschirmen. Die Abschirmungen können durch ein beliebiges geeignetes Material, wie z. B. Stahl oder Blei implementiert sein. Bei Ausführungsbeispielen der Erfindung sind die Abschirmungen aus Bleiplatten implementiert, da die Platten dann mit einer wesentlich geringeren Dicke ausgeführt sein können, was im Endeffekt zu einem geringeren Gewicht führt.

Erfindungsgemäß sind somit nur die Teile der Computertomographievorrichtung, in denen eine Röntgenstrahlung auftritt, mit einer Abschirmung versehen, d. h. nur Komponenten, die Röntgenstrahlung ausgesetzt sind, sind in einem abgeschirmten Raum angeordnet. Gemäß den Figuren 1a und 1b sind separate Abschirmungen 12 und 14 vorgesehen. Es ist jedoch offensichtlich, dass bei alternativen Ausführungsbeispielen eine einzige Abschirmung oder eine größere Anzahl von Abschirmungen vorgesehen sein kann. Dadurch, dass erfindungsgemäß nur Komponenten, die einer Röntgenstrahlung ausgesetzt sind, in dem abgeschirmten Raum angeordnet sind, kann bei Ausführungsbeispielen eine optimale Gewichtsreduzierung erhalten werden. An der Unterseite kann, wie dargestellt ist, zumindest teilweise eine Abschirmung vorgesehen sein, um zu verhindern, dass Strahlung nach unten austritt. Bei Ausführungsbeispielen der Erfindung kann Sicherheitselektronik hinter der Abschirmung angeordnet sein.

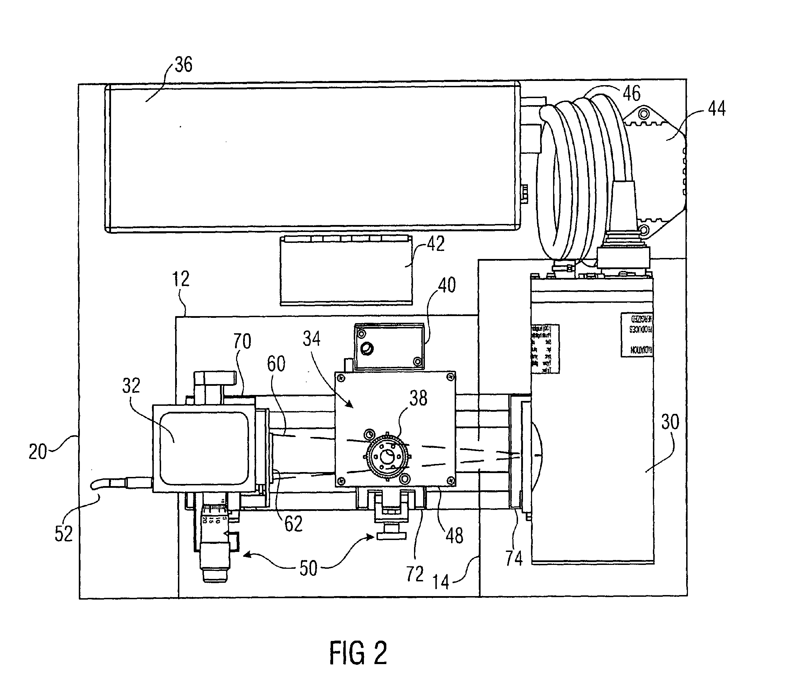

Fig. 2 zeigt eine schematische Draufsicht auf eine Computertomographievorrichtung, wobei ein tragbares Gehäuse 20 schematisch durch ein Rechteck dargestellt ist. Das Gehäuse weist Abmessungen auf, die es einer durchschnittliche Person ermöglichen, dasselbe zu tragen. Ferner weist das Gehäuse samt Inhalt ein Gewicht auf, das es einer durchschnittlichen Person ermöglicht, es zu tragen, beispielsweise ein maximales Gewicht von 25 kg.

In dem tragbaren Gehäuse 20 sind Komponenten der Computertomographievorrichtung angeordnet, die ausgebildet sind, um Signale zu erzeugen, die eine computertomographische Darstellung eines Objekts ermöglichen. Diese Komponenten umfassen eine Röntgenstrahlungsquelle 30, einen Röntgenstrahlungsdetektor 32, einen Objektraum 34, einen Hochspannungsgenerator 36, eine Rotationseinrichtung 38, eine Verfahreinrichtung 40, ein Steuergerät 42 für die Rotationseinrichtung 38 und ein Steuergerät 44 für die Verfahreinrichtung 40. Die Röntgenstrahlungsquelle 30 ist über ein Hochspannungskabel 46 mit dem Hochspannungsgenerator 36 verbunden.

Die Rotationseinrichtung 38 ist auf einem Drehtisch 48 angeordnet. Justiervorrichtungen 50 zum manuellen Einstellen der Positionen des Röntgenstrahlendetektors 32 und des Drehtisches 48 können vorgesehen sein.

Die Hochspannungsgenerator 36 kann ausgebildet sein, um eine übliche Netzspannung (beispielsweise 220 V) zu empfangen und diese in die für die Röntgenstrahlungsquelle 30 erforderliche Hochspannung umzuwandeln. Zu diesem Zweck kann in üblicher Weise ein Anschluss an ein externes Spannungsversorgungsnetz vorgesehen sein. Entsprechende Generatoren sind Fachleuten bekannt. Die Computertomographievorrichtung kann weiterhin entsprechende Netzteile (nicht gezeigt) aufweisen, um die für die übrigen Komponenten erforderlichen Versorgungsspannungen zu erzeugen. Neben dem Hochspannungskabel 46 erforderliche elektrische Verbindungen, beispielsweise zwischen den Steuereinrichtungen 42 und 44 und der Rotationseinrichtung 38 bzw. der Verfahreinrichtung 40 sind aus Übersichtlichkeitsgründen nicht dargestellt.

Die von dem Röntgenstrahlungsdetektor 32 erzeugten Signale können beispielsweise nach einer Vorverarbeitung derselben über eine USB-Schnittstelle an einen Computer ausgegeben werden, wobei eine solche USB-Schnittstelle schematisch bei 52 gezeigt ist. Bei Ausführungsbeispielen kann die Computertomographievorrichtung ausgebildet sein, um über den USB-Anschluss von extern durch einen Computer gesteuert zu werden. Dabei können sämtliche Komponenten der Computertomographievorrichtung, wie z.B. Hochspannungsgenerator, Verfahreinrichtung, Rotationseinrichtung und Detektor über die USB-Schnittstelle gesteuert werden.

Ferner sind in Fig. 2 die Abschirmungen 12 und 14 schematisch dargestellt. Wie in Fig. 2 gezeigt ist, ist bei dem dargestellten Ausführungsbeispiel der Röntgenstrahlungsdetektor 32 nicht vollständig in der Abschirmung 12 angeordnet. Bei alternativen Ausführungsbeispielen kann der Röntgenstrahlungsdetektor 32 vollständig in der Abschirmung angeordnet sein.

Im Betrieb erzeugt der Generator 36 die für die Röntgenstrahlungsquelle 30 erforderliche Hochspannung, die über das Hochspannungskabel 46 zu der Röntgenstrahlungsquelle 30 übertragen wird. Die Röntgenstrahlungsquelle 30 erzeugt einen Röntgenstrahl 60, der auf eine Detektorfläche 62 des Röntgenstrahlungsdetektors 32 fällt. Ein Objekt wird auf der Rotationseinrichtung 38 platziert. Die Rotationseinrichtung 38 und somit das Objekt drehen sich unter Steuerung des Steuergeräts 42. Der zu untersuchende Bereich des Objekts (nicht dargestellt) befindet sich dabei in horizontaler Richtung vollständig in dem Röntgenstrahl 60. Der Röntgenstrahlungsdetektor erfasst die zur Erzeugung einer Computertomographie-Darstellung erforderlichen Signale und gibt diese über die USB-Schnittstelle 52 aus.

Befindet sich das Objekt aufgrund der fächerförmigen Ausbildung des Röntgenstrahls auch in vertikaler Richtung vollständig im Röntgenstrahl, so reicht die Rotationseinrichtung aus, um eine vollständige computertomographische Darstellung zu erzeugen. Besitzt das Objekt in vertikaler Richtung eine größere Abmessung als die Erstreckung des Röntgenstrahls, so wird der Drehtisch 48 mit der darauf angeordneten Rotationseinrichtung 38 durch die Verfahreinrichtung 40, die durch die Verfahreinrichtungssteuerung 44 entsprechend gesteuert wird, in vertikaler Richtung bewegt, so dass eine vollständige computertomographische Aufnahme des Objekts erreicht werden kann. Durch die Verfahreinrichtung 40, die auch als Hubachse bezeichnet werden kann, und die Rotationseinrichtung 38, die auch als Rotationsachse bezeichnet werden kann, ist es somit möglich, eine Helix-Computertomographie durchzuführen und Objekte zu untersuchen, die größer sind als der Detektor.

Erfindungsgemäße Ausführungsbeispiele umfassen somit eine Positionierungseinrichtung, die durch die Rotationseinrichtung 38 und/oder die Verfahreinrichtung 40 gebildet ist.

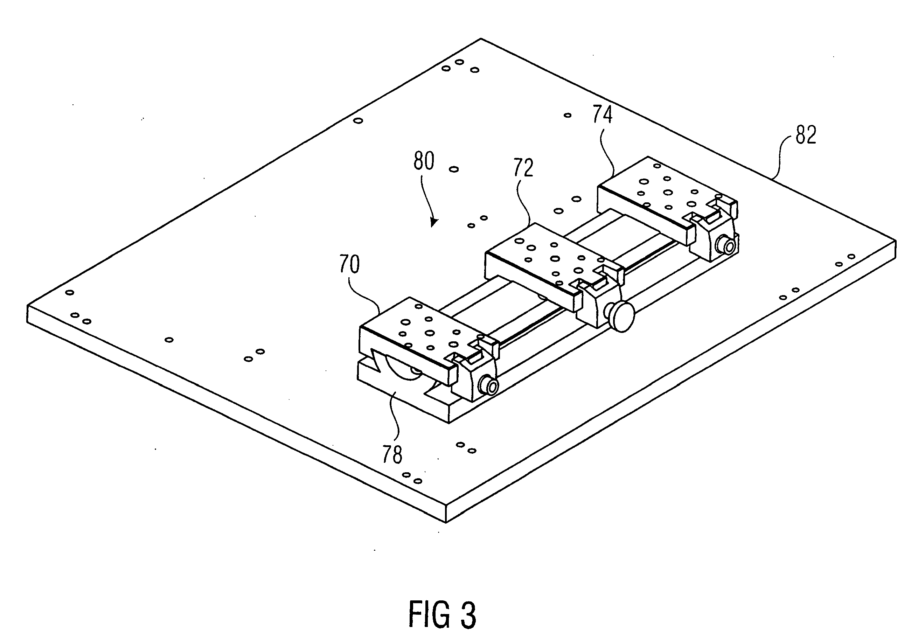

In Fig. 2 sind schematisch ferner Befestigungsmittel 70 für den Röntgenstrahlungsdetektor 32, Befestigungsmittel 72 für den Drehtisch 48 und Befestigungsmittel 74 für die Röntgenstrahlungsquelle 30 dargestellt. Diese Befestigungsmittel werden nun Bezug nehmend auf Fig. 3 näher erläutert.

Wie in Fig. 3 gezeigt ist, können die Befestigungsmittel 70, 72 und 74 durch jeweilige Platten gebildet sein, die an einer Schiene 78 befestigt sind. Die Befestigungsplatten 70, 72 und 74 können schiebbar und arretierbar an der Schiene 78 befestigt sein. Die Befestigungsplatten 70, 72 und 74 und die Schiene 78 stellen ein Schienensystem 80 dar. Wie in Fig. 3 gezeigt ist, ist das Schienensystem 80 an einer Bodenplatte 82 der Computertomographievorrichtung befestigt. Durch die Befestigung von Röntgenstrahlungsquelle, die durch eine übliche Röntgenröhre gebildet sein kann, Röntgenstrahlungsdetektor und Objekt auf einem einzigen Schienensystem 80 kann eine hohe Steifigkeit erreicht werden. Die Teile des Schienensystems können beispielsweise aus Aluminium gebildet sein.

Bei Ausführungsbeispielen der Erfindung kann die Bodenplatte 82 in Sandwich-Bauweise ausgeführt sein, so dass die Steifigkeit der Bodenplatte weiter erhöht und gleichzeitig Gewicht gespart werden kann. Die in Sandwich-Bauweise ausgeführte Bodenplatte kann beispielsweise durch eine in Wabenstruktur aufgebaute Aluminium-Sandwich-Bodenplatte ausgebildet sein.

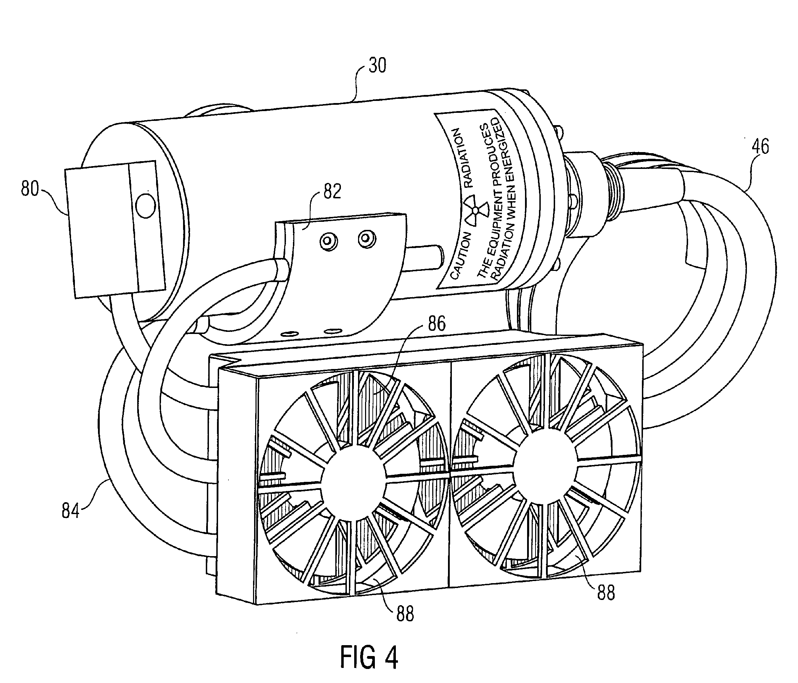

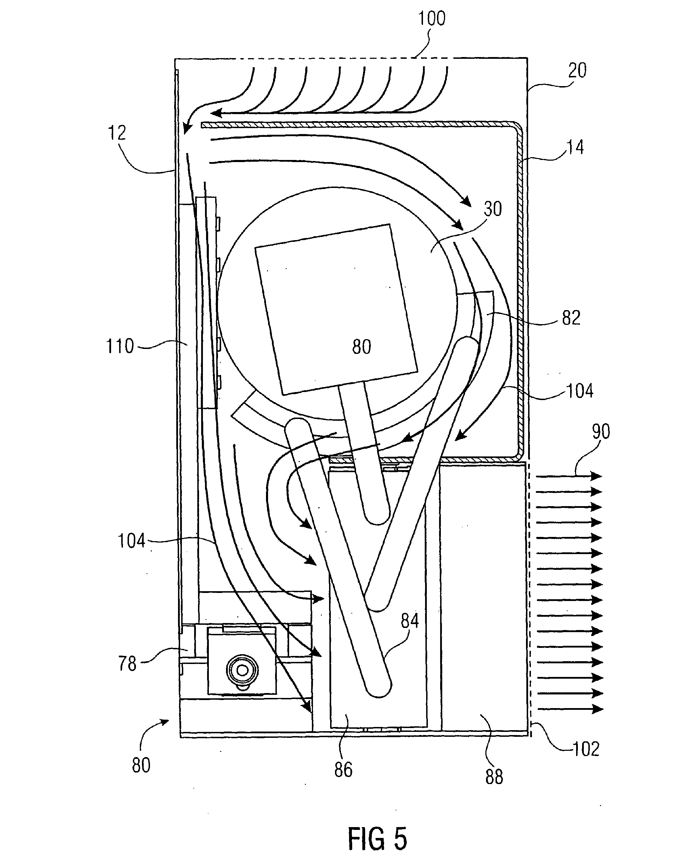

Nachfolgend wird Bezug nehmend auf die Figuren 4 und 5 eine Kühleinrichtung (in Fig. 2 aus Übersichtlichkeitsgründen nicht gezeigt) für die Röntgenstrahlungsquelle 30 beschrieben. Wie in Fig. 4 gezeigt ist, weist die Kühleinrichtung Wärmekoppelelemente 80 und 82 auf, die an der Röntgenstrahlungsquelle 30 angebracht sind. Wärmeleitungen 84, sogenannte Heat-Pipes, sind thermisch mit den Wärmekoppelelementen 82 gekoppelt und leiten die Wärme an Kühllamellen 86 ab. Die Wärmeleitungen 84 können beispielsweise durch hohle Leitungen, die mit einer Kühlflüssigkeit gefüllt sind, implementiert sein. Ein oder mehrere Lüfter 88 sind vorgesehen, um die zu den Kühllamellen transportierte Wärme aus dem Gehäuse zu entfernen, wie durch Pfeile 90 in Fig. 5 gezeigt ist. Wie ferner in Fig. 5 gezeigt ist, weist das dort abschnittsweise gezeigte Gehäuse 20 obere Entlüftungsöffnungen 100 und seitliche Entlüftungsöffnungen 102 auf. Zwischen den Entlüftungsöffnungen 100 und 102 existiert ein Windkanal, der teilweise durch die Abschirmungen 12, 14 begrenzt wird, und der sich von den oberen Lüftungsöffnungen 100 zu den Kühllamellen 86 erstreckt, wie durch Pfeile 104 in Fig. 5 gezeigt ist. Durch den oder die Lüfter 88 wird eine Strömung entlang dieses Windkanals erzeugt, so dass dadurch die Röntgenstrahlungsquelle 30 gekühlt und die Wärme von den Lamellen 86 nach außen transportiert werden kann, wie durch die Pfeile 90 gezeigt ist. Dadurch ist es möglich, eine Kühlung der Röntgenstrahlungsquelle bei einem sehr geringen Raumbedarf zu implementieren, was wiederum eine Erhöhung der Lebensdauer zur Folge hat.

In Fig. 5 ist ferner schematisch eine Halterung 110 dargestellt, über die die Röntgenstrahlungsquelle 30 an der Schiene 78 des Schienensystems 80 angebracht ist.

Ausführungsbeispiele der vorliegenden Erfindung umfassen somit ein ausgeklügeltes Kühlsystem für die Röntgenstrahlungsquelle, d. h. die Röhre, das eine kompakte Bauweise und eine Erhöhung der Lebensdauer ermöglicht. Wie Bezug nehmend auf Fig. 3 erläutert wurde, umfassen Ausführungsbeispiele der vorliegenden Erfindung eine Kombination einer Leichtbau-Wabensandwichplatte und eines Schienensystems für eine optimale Steifigkeit.

Ausführungsbeispiele der Erfindung ermöglichen durch die Kombination einer kleinen Röntgenstrahlungsquelle, eines kompakten Detektors, kompakter Positionierungseinrichtungen, wie Rotationseinrichtung und Hubeinrichtung, und die Auswahl möglichst kleiner Steuergeräte eine vorteilhafte Anordnung aller Komponenten, die eine Minimierung des Volumens der Vorrichtung ermöglicht. Röntgenstrahlungsquelle und Hochspannungsgenerator dafür können in einem Winkel von 90° zueinander angeordnet sein. Die übrigen Komponenten können wie in Fig. 2 dargestellt angeordnet sein, um eine möglichst Platz sparende Anordnung zu erhalten. Selbstverständlich kann bei alternativen Ausführungsbeispielen eine abweichende Anordnung implementiert werden, solange ein geringes Volumen, das eine Tragbarkeit ermöglicht, erhalten bleibt.

Wie oben ausgeführt wurde, umfassen Ausführungsbeispiele der Erfindung sowohl eine Rotationseinrichtung als auch eine Verfahreinrichtung. Dadurch wird eine Helix-Computertomographie ermöglicht, so dass es möglich ist, Bauteile zu untersuchen, die größer sind als der Detektor selbst. Ferner kann durch eine solche Vorgehensweise die Qualität der Rekonstruktionen durch Vermeidung von Artefakten deutlich verbessert werden. Weiterhin ermöglicht eine Helix-Computertomographie ein Vermessen von Objekten.

Bei Ausführungsbeispielen der Erfindung kann die Steuerung über einen einzigen USB-Anschluss erfolgen, so dass ein kompliziertes Anschließen der Anlage an einen PC entfallen kann. Ausführungsbeispiele der Erfindung weisen somit einen universellen Anschluss auf, der jeweils an den aktuellen Stand der Technik angepasst sein kann, um den Einsatz nahezu jedes PCs oder Notebooks zu ermöglichen, da dieselben entsprechende Anschlussmöglichkeiten aufweisen. Bei Ausführungsbeispielen der Erfindung können daher nur zwei externe Anschlüsse ausreichend sein, ein Anschluss an ein externes Spannungsversorgungsnetz und ein Anschluss zur Ausgabe der erzeugten Signale, der gleichzeitig als Steueranschluss dienen kann.

Die vorliegende Erfindung ermöglicht somit die Implementierung einer tragbaren Computertomographievorrichtung, und insbesondere einer tragbaren Computertomographievorrichtung mit einem Gewicht von weniger als 25 kg oder sogar weniger als 20 kg. Somit darf die Vorrichtung von einer Person getragen werden und kann somit überall eingesetzt werden. Es bedarf keiner weiteren Erläuterung, dass das tragbare Gehäuse mit geeigneten Mitteln versehen sein kann, um die Tragbarkeit desselben zu unterstützen, wie z.B. Griffen, Grifföffnungen oder Griffeinbuchtungen.

Ausführungsbeispiele der Erfindung schaffen eine Computertomographievorrichtung mit einem von einer Person tragbaren Gehäuse, einer Mehrzahl von in dem Gehäuse angeordneten Komponenten, die ausgebildet sind, um Signale zu erzeugen, die eine computertomographische Darstellung eines Objekts ermöglichen, und einer Abschirmungseinrichtung, die einen abgeschirmten Raum in dem tragbaren Gehäuse definiert, um die Umgebung vor in dem tragbaren Gehäuse erzeugter Röntgenstrahlung abzuschirmen, wobei die Komponenten, die Röntgenstrahlung ausgesetzt sind, in dem abgeschirmten Raum angeordnet sind, und wobei zumindest eine der Komponenten außerhalb des abgeschirmten Raums in dem Gehäuse angeordnet ist.

Bei Ausführungsbeispielen können die Komponenten eine Röntgenstrahlungsquelle, einen Röntgenstrahlungsdetektor, einen Objektraum zwischen der Röntgenstrahlungsquelle und dem Röntgenstrahlungsdetektor und einen Hochspannungsgenerator aufweisen, wobei die Röntgenstrahlungsquelle, der Objektraum und zumindest teilweise der Röntgenstrahlungsdetektor in dem abgeschirmte Raum angeordnet sind, und der Hochspannungsgenerator außerhalb des abgeschirmten Raums angeordnet ist. Dabei können die Röntgenstrahlungsquelle und der Hochspannungsgenerator jeweils eine Längsachse aufweisen, wobei die Röntgenstrahlungsquelle und der Hochspannungsgenerator derart in dem Gehäuse angeordnet sind, dass die Längsachsen senkrecht zueinander sind. Eine Einrichtung zum Kühlen der Röntgenstrahlungsquelle kann bei Ausführungsbeispielen vorgesehen sein. Die Einrichtung zum Kühlen kann Wärmekoppelelemente, die an der Röntgenstrahlungsquelle angebracht sind, Wärmeleitungen zum Ableiten von Wärme von dem Wärmekoppelelement zu Kühllamellen und zumindest einen Lüfter, um Wärme von den Kühllamellen aus dem Gehäuse zu transportieren, aufweisen. Bei Ausführungsbeispielen können die Komponenten eine Positionierungseinrichtung zum Positionieren eines Objekts in dem Objektraum aufweisen. Die Positionierungseinrichtung kann eine Rotationseinrichtung aufweisen, die ausgelegt ist, um ein Objekt relativ zu der durch die Röntgenstrahlungsquelle erzeugten Strahlung zu drehen, und/oder eine Verfahreinrichtung aufweisen, die ausgelegt ist, um das Objekt relativ zu der Röntgenstrahlung zu verfahren, um verschiedene Abschnitte des Objekts der Röntgenstrahlung auszusetzen. Bei Ausführungsbeispielen können die Komponenten zumindest eine Steuereinrichtung für die Positionierungseinrichtung aufweisen, wobei die Steuereinrichtung außerhalb des abgeschirmten Raums in dem Gehäuse angeordnet ist.

Bei Ausführungsbeispielen können die Komponenten elektronische Bauteile aufweisen, die außerhalb des abgeschirmten Raums in dem Gehäuse angeordnet sind. Bei Ausführungsbeispielen kann das Gehäuse kann eine Bodenplatte aufweisen, die in Sandwich-Bauweise ausgeführt ist. Bei Ausführungsbeispielen kann die Computertomographievorrichtung ein Schienensystem aufweisen, an dem zumindest einige der Komponenten befestigt sind. Bei Ausführungsbeispielen können die Röntgenstrahlungsquelle, die Positionierungseinrichtung und der Röntgenstrahlungsdetektor an dem Schienensystem befestigt sein. Bei Ausführungsbeispielen kann die Computertomographievorrichtung einen USB-Anschluss aufweisen. Bei Ausführungsbeispielen kann die Computertomographievorrichtung ein Gesamtgewicht von maximal 25 kg oder maximal 20 kg aufweisen und äußere Abmessungen von maximal 350 mm x 300 mm x 230 mm aufweisen.

## Patentansprüche

1. Computertomographievorrichtung mit folgenden Merkmalen:
einem von einer Person tragbaren Gehäuse (20);
einer Mehrzahl von in dem Gehäuse (20) angeordneten Komponenten (30-44), die ausgebildet sind, um Signale zu erzeugen, die eine computertomographische Darstellung eines Objekts ermöglichen; und
eine Abschirmungseinrichtung (12, 14), die einen abgeschirmten Raum in dem tragbaren Gehäuse (20) definiert, um die Umgebung vor in dem tragbaren Gehäuse (20) erzeugter Röntgenstrahlung (60) abzuschirmen,
wobei Komponenten (30, 32, 34) der Mehrzahl von in dem Gehäuse angeordneten Komponenten, die Röntgenstrahlung ausgesetzt sind, in dem abgeschirmten Raum angeordnet sind, und wobei zumindest eine der Komponenten (36, 42, 44) außerhalb des abgeschirmten Raums in dem Gehäuse (20) angeordnet ist,
wobei die Komponenten eine Röntgenstrahlungsquelle (30), einen Röntgenstrahlungsdetektor (32), einen Objektraum (34) zwischen der Röntgenstrahlungsquelle (30) und dem Röntgenstrahlungsdetektor (32) und einen Hochspannungsgenerator (36) aufweisen, wobei die Röntgenstrahlungsquelle (30), der Objektraum (34) und zumindest teilweise der Röntgenstrahlungsdetektor (32) in dem abgeschirmte Raum angeordnet sind, und der Hochspannungsgenerator (36) außerhalb des abgeschirmten Raums angeordnet ist, und
wobei die Komponenten eine Positionierungseinrichtung (38, 40) zum Positionieren eines Objekts in dem Objektraum (34) und zumindest eine Steuereinrichtung (42, 44) für die Positionierungseinrichtung aufweisen, wobei die Steuereinrichtung (42, 44) außerhalb des abgeschirmten Raums in dem Gehäuse (20) angeordnet ist,
wobei Computertomographievorrichtung ein Gesamtgewicht von maximal 25 kg und äußere Abmessungen von maximal 350 mm x 300 mm x 230 mm aufweist,
wobei die Röntgenstrahlungsquelle (30) und der Hochspannungsgenerator (36) jeweils eine Längsachse aufweisen, wobei die Röntgenstrahlungsquelle (30) und der Hochspannungsgenerator (36) derart in dem Gehäuse (20) angeordnet sind, dass die Längsachsen senkrecht zueinander sind,
wobei die Positionierungseinrichtung eine Verfahreinrichtung (40) aufweist, die ausgelegt ist, um das Objekt relativ zu der Röntgenstrahlung (60) zu verfahren, um verschiedene Abschnitte des Objekts der Röntgenstrahlung auszusetzen,
wobei das Gehäuse (20) eine Bodenplatte (82) aufweist, die in Sandwich-Bauweise ausgeführt ist,
wobei die Computertomographievorrichtung ein Schienensystem (80) aufweist, an dem zumindest einige der Komponenten befestigt sind, und
wobei die Röntgenstrahlungsquelle (30), die Positionierungseinrichtung (38, 40) und der Röntgenstrahlungsdetektor (32) an dem Schienensystem (80) befestigt sind.

2. Computertomographievorrichtung nach Anspruch 1, die eine Einrichtung zum Kühlen der Röntgenstrahlungsquelle (30) aufweist.

3. Computertomographievorrichtung nach Anspruch 2, bei der die Einrichtung zum Kühlen Wärmekoppelelemente (80, 82), die an der Röntgenstrahlungsquelle (30) angebracht sind, Wärmeleitungen (84) zum Ableiten von Wärme von dem Wärmekoppelelement (80, 82) zu Kühllamellen (86) und zumindest einen Lüfter (88), um Wärme von den Kühllamellen (86) aus dem Gehäuse (20) zu transportieren, aufweist.

4. Computertomographievorrichtung nach einem der Ansprüche 1 bis 3, bei der die Positionierungseinrichtung (38, 40) eine Rotationseinrichtung (38) aufweist, die ausgelegt ist, um ein Objekt relativ zu der durch die Röntgenstrahlungsquelle (30) erzeugten Strahlung (60) zu drehen.

5. Computertomographievorrichtung nach einem der Ansprüche 1 bis 4, bei der die Komponenten elektronische Bauteile aufweisen, die außerhalb des abgeschirmten Raums in dem Gehäuse (20) angeordnet sind.

6. Computertomographievorrichtung nach einem der Ansprüche 1 bis 5, die einen USB-Anschluss (52) aufweist.

## Claims

1. Computer tomography device, comprising:
a casing (20) portable by a person;
a plurality of components (30 to 44) arranged in the casing (20), which are implemented to generate signals allowing computer tomographical illustration of an object; and
shielding means (12, 14) defining a shielded space in the portable casing (20) in order to shield the environment from X-radiation (60) generated in the portable casing (20),
wherein components (30, 32, 34) of the plurality of components arranged in the casing that are subject to X-radiation are arranged in the shielded space, and
wherein at least one of the components (36, 42, 44) is arranged outside the shielded space in the casing (20),
wherein the components comprise an X-ray source (30), an X-ray detector (32), an object space (34) between the X-ray source (30) and the X-ray detector (32) and a high-voltage generator (36), wherein the X-ray source (30), the object space (34) and at least partly the X-ray detector (32) are arranged in the shielded space, and
the high-voltage generator (36) is arranged outside the shielded space, and
wherein the components comprise a positioning means (38, 40) for positioning an object in the object space (34) and at least one control means (42, 44) for the positioning means, wherein the control means (42, 44) is arranged outside the shielded space in the casing (20),
wherein the computer tomography device has an overall weight of a maximum of 25 kg and external dimensions of a maximum of 350 mm x 300 mm x 230 mm,
wherein the X-ray source (30) and the high-voltage generator (36) each comprise longitudinal axes, wherein the X-ray source (30) and the high-voltage generator (36) are arranged in the housing (20) such that the longitudinal axes are perpendicular to one another,
wherein the positioning means comprises a moving means (40), which is implemented to move the object relative to the X-radiation (60) in order to subject different sections of the object to the X-radiation,
wherein the casing (20) comprises a floor plate (82) which is implemented in sandwich construction,
wherein the computer tomography device comprises a rail system (80) on which at least some of the components are mounted, and
wherein the X-ray source (30), the positioning means (38, 40) and the X-ray detector (32) are mounted on the rail system (80).

2. Computer tomography device according to claim 1, comprising a means for cooling the X-ray source (30).

3. Computer tomography device according to claim 2, wherein the means for cooling comprises heat coupling elements (80, 82) that are mounted on the X-ray source (30), heat lines (84) for deviating heat from the heat coupling elements (80, 82) to cooling fins (86) and at least one fan (88) in order to transport heat from the cooling fins (86) out of the casing (20).

4. Computer tomography device according to one of claims 1 to 3, wherein the positioning means (38, 40) comprises a rotation means (38) which is implemented to rotate an object relative to the radiation (60) generated by the X-ray source (30).

5. Computer tomography device according to one of claims 1 to 4, wherein the components comprise electronic members that are arranged outside the shielded space in the casing (20).

6. Computer tomography device according to one of claims 1 to 5, comprising a USB terminal (52).

## Revendications

1. Dispositif de tomographie assistée par ordinateur, aux caractéristiques suivantes:
un boîtier (20) portable par une personne;
une pluralité de composants (30 à 44) disposés dans le boîtier (20), lesquels sont réalisés pour générer des signaux qui permettent une représentation tomographique assistée par ordinateur d'un objet; et
un moyen de blindage (12, 14) définissant un espace blindé dans le boîtier portable (20) destiné à protéger l'environnement contre le rayonnement X (60) généré dans le boîtier portable (20),
dans lequel les composants (30, 32, 34) parmi la pluralité de composants disposés dans le boîtier qui sont exposés aux rayons X sont disposés dans l'espace blindé, et dans lequel au moins l'un des composants (36, 42, 44) est disposé à l'extérieur de l'espace blindé dans le boîtier (20),
dans lequel les composants présentent une source de rayonnement X (30), un détecteur de rayonnement X (32), un espace à objet (34) entre la source de rayonnement X (30) et le détecteur de rayonnement X (32) et un générateur de haute tension (36), dans lequel la source de rayonnement X (30), l'espace à objet (34) et au moins partiellement le détecteur de rayonnement X (32) sont disposés dans l'espace blindé, et le générateur de haute tension (36) est disposé en dehors de l'espace blindé, et
dans lequel les composants présentent un moyen de positionnement (38, 40) destiné à positionner un objet dans l'espace à objet (34) et au moins un moyen de commande (42, 44) du moyen de positionnement, dans lequel le moyen de commande (42, 44) est disposé en dehors de l'espace protégé dans le boîtier (20),
dans lequel le dispositif de tomographie assistée par ordinateur présente un poids total de tout au plus 25 kg et des dimensions extérieures de tout au plus 350 mm x 300 mm x 230 mm,
dans lequel la source de rayonnement X (30) et le générateur de haute tension (36) présentent, chacun, un axe longitudinal, dans lequel la source de rayonnement X (30) et le générateur de haute tension (36) sont disposés dans le boîtier (20) de sorte que les axes longitudinaux soient perpendiculaires l'un à l'autre,
dans lequel le moyen de positionnement présente un moyen de déplacement (40) qui est conçu pour déplacer l'objet par rapport au rayonnement X (60), pour exposer différents segments de l'objet au rayonnement X,
dans lequel le boîtier (20) présente une plaque de fond (82) qui est réalisée selon la construction en sandwich,
dans lequel le dispositif de tomographie assistée par ordinateur présente un système de rails (80) auquel sont fixés au moins quelques-uns des composants, et
dans lequel la source de rayonnement X (30), le moyen de positionnement (38, 40) et le détecteur de rayonnement X (32) sont fixés au système de rails (80).

2. Dispositif de tomographie assistée par ordinateur selon la revendication 1, présentant un moyen pour refroidir la source de rayonnement X (30).

3. Dispositif de tomographie assistée par ordinateur selon la revendication 2, dans lequel le moyen destiné à refroidir présente des éléments de couplage thermique (80, 82) qui sont placés sur la source de rayonnement X (30), des conduits de chaleur (84) destinés à dériver la chaleur provenant de l'élément de couplage thermique (80, 82) vers des ailettes de refroidissement (86) et au moins un ventilateur (88), pour transporter la chaleur des ailettes de refroidissement (86) hors du boîtier (20).

4. Dispositif de tomographie assistée par ordinateur selon l'une des revendications 1 à 3, dans lequel le moyen de positionnement (38, 40) présente un moyen de rotation (38) qui est conçu pour faire tourner un objet par rapport au rayonnement (60) généré par la source de rayonnement X (30).

5. Dispositif de tomographie assistée par ordinateur selon l'une des revendications 1 à 4, dans lequel les composants présentent des composants électroniques qui sont disposés en dehors de l'espace blindé dans le boîtier (20).

6. Dispositif de tomographie assistée par ordinateur selon l'une des revendications 1 à 5, qui présente une connexion USB (52).
